# EUROPEAN PATENT APPLICATION

(11) **EP 1 958 624 A1**
(43) Date of publication of application: **20.08.2008**
(21) Application number: 07003315.4
(22) Date of filing: 16.02.2007
(51) Int. Cl.: A61K 9/51, A61K 47/48, A61K 33/30, A61K 33/34, C08L 101/02

(54) **Divalent metal-ion loaded nano-transport system having a dendritic architecture useful for therapy**

(71) Applicant: Freie Universität Berlin, 14195 Berlin (DE)
(72) Inventor: Multhaup, Gerd, Prof., Dr., 14532 Kleinmachnow (DE); Haag, Rainer, Prof., Dr., 12209 Berlin (DE); Treiber, Carina, Dr., 14195 Berlin (DE)
(74) Representative: Schüssler, Andrea

(57) **Abstract**

Described is the use of a nanocarrier having a dendritic structure which is composed of a dendritic core and at least one shell for the non-covalent encapsulation and/or transport of divalent metal-ions, preferably Cu-ions or Zn(II)-ions. Also described is a pharmaceutical composition containing such nanocarrier complexed divalent metal-ions in a non-covalent encapsulated form. Finally several therapeutic uses of such a nanocarrier divalent metal-ion complex are described, for example for slowing down ageing or for the treatment of Cu-ion transport disorders like Alzheimer's disease (AD) or Menkes disease.

## Description

The present invention relates to the use of a nanocarrier having a dendritic structure which is composed of a dendritic core and at least two shells for the non-covalent encapsulation and/or transport of divalent metal-ions, preferably Cu- or Zn(II)-ions. The present invention also relates to a pharmaceutical composition containing such a nanocarrier carrying divalent metal-ions like Cu-ions or Zn(II)-ions in a non-covalent encapsulated form. Moreover, the present invention relates to several therapeutic uses of such a nanocarrier carrying divalent metal-ions in a non-covalent encapsulated form, for example for the therapy of Alzheimer's disease (AD) or Menkes disease.

The generation of nanocompartments for the homogeneous complexation and dissolution of active agents was an unsolved problem for many applications, for example, in catalysis, drug delivery, and ink formulation, among others. Supramolecular transport systems can be divided into two classes: (a) physical aggregates of amphiphilic molecules such as vesicles or micelles and (b) covalently connected molecular architectures, so-called unimolecular transport systems. A fundamental problem of all these carrier systems, however, was their limited matrix compatibility. They can either transport nonpolar molecules into an aqueous environment or, if the system relies on an inverted micellar architecture, transfer polar molecules into a hydrophobic environment, such as an organic medium.

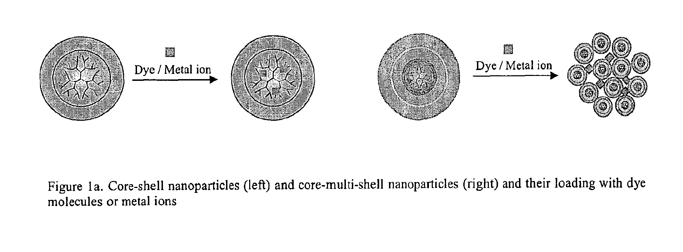

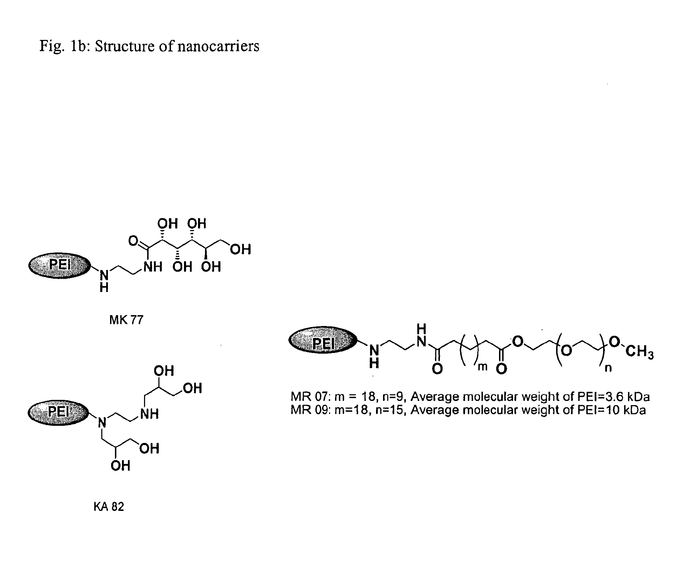

The generation of nanocompartments that are compatible with various environments solved many solubility and stability problems of active agents. Thus, engineered nanoparticles, particularly those with core/shell architecture have found potential applications in the field of biology and therapeutics. These structures are formed by covalent modification of dendritic macromolecules with an appropriate shell that results in stable micelle-type structures (Haag, Angew. Chem. 116 (2004), 280-4; Haag, Angew. Chem. Int. Ed. 43 (2004), 278-82). These molecular architectures are suitable for the non-covalent encapsulation of guest molecules ranging from ionic to molecular dimension. Recently, a simple and general concept for the generation of core-shell-type architecture from readily accessible starting materials with an aim to deliver drug and related bioactive molecules to target tissues has been described (WO 2006/018295; Krämer et al., Macromolecules 38 (2005), 8308-15; Radowski et al., Angew. Chem. 119 (2007), 1287-1292. Two types of architectures have been devised involving either one shell (core-shell nanoparticles, CS NP) or multiple shells (core-multi-shell nanoparticles, CMS NP) attached to a dendritic polyamine core as illustrated in Figure 1. In case of CMS NP, alkyl chain and polyethylene glycol (PEGs) of different chain length have been used as the inner and outer shell respectively. These two types of nanoparticles, though different in their structure, are comparable in size and both show strong affinity to complex with wide range of guest molecules.

A disturbed metal-ion homeostasis has been described during ageing in mice and men. In addition, it was found that the pathogenesis of Alzheimer's disease (AD) is associated with Cu-depletion of the brain. There are several other human diseases associated with neurodegenerative symptoms, such as amyotrophic lateral sclerosis (ALS), Creutzfeld Jakob disease (CJD), Parkinson's disease and Cu transport disorders, i.e.

Wilson and Menkes disease. The transport and cellular metabolism of Cu depends on a series of membrane proteins and smaller soluble proteins that comprise a functionally integrated system for maintaining cellular Cu-homeostasis. However, so far the treatment of such diseases by application of Cu-ions was very limited, e.g., due to solubility problems.

Therefore, it is the object of the present invention to provide a means allowing to efficiently deliver metal-ions like Zn(II)-ions and Cu-ions to the desired target organ or tissue.

According to the invention this is achieved by the subject matters defined in the claims.

During the experiments leading to the present invention it was found that some nanocarriers offer a possibility to bypass the cellular metal-ion (e.g., Cu) uptake system to achieve higher local concentrations compared to normal cellular uptake to rescue not only the effects of Cu deficiency of certain diseases such as AD but also deficiencies of other divalent metal-ions, e.g., Zn(II) in specific tissues or cell types.

The transport and cellular metabolism of Cu normally depends on a series of membrane proteins and smaller soluble cytoplasmic proteins that comprise a functionally integrated system for maintaining cellular Cu homeostasis. The normal pathway of Cu uptake involves a reduction of Cu(II) to Cu(I) prior to the transfer across the membrane (Aller and Unger, Proc. Natl. Acad. Sci. USA 103 (2006), 3627-32). Excess copper is highly toxic by causing oxidative stress via Fenton reaction and thereby damages proteins, DNA and lipids. The nanocarriers of the present invention will not only provide an excellent system to study the import of Cu into the cell by bypassing the normal cellular mechanisms but also the effect of Cu in specific cellular compartments by targeting individual compartments with Cu-loaded carriers. Specific Cu-delivery across cell membranes to intracellular target- and storage sites could be demonstrated. Thus, the transport of Cu-carriers across the blood-brain barrier will be achieved with the nanocarriers, e.g., to rescue Cu-deficiency in AD brain.

Taken together, nanocarriers that specifically transport their cargo across cell membranes and to specific cellular substructures were identified. These data uncover a novel biological function for nanotransporters in cellular transport of metal-ions.

Accordingly, the present invention relates to the use of a nanocarrier having a dendritic structure which is composed of a dendritic core and at least one, preferably at least two shells for the non-covalent encapsulation and/or transport of divalent metal-ions, preferably Cu-ions or Zn(II)-ions.

The "dendritic structure" is achieved by the use of dendritic polymers. Such dendritic and methods for producing these compounds are, e.g., described in Haag, Angew. Chem. 119(2007) 1287-1292; Haag, Angew. Chem. 114 (2002), 4426-31; WO 02/077037; WO 03/037383 and US 2002/0187199.

Methods for preparing nanocarriers are, e.g., described in Example 1, below, and WO 2006/018295. These documents also describe nanoparticles having a structure which is particularly useful for the purposes of the present invention.

Loading of the nanocarriers with metal-ions can be carried by routine methods; see also Example 1, below. Preferably, the nanoparticles are produced by a modular approach using for assembly cheap, commercially available building blocks, e.g., hyperbranched poly(ethylene imine) (PEI), dicarboxylic acids and monomethyl poly(ethylene glycol) (mPEG). The hyperbranched cores can be functionalized with linear amphiphilic building blocks formed by alkyl acids (e.g., C₆, C₁₂ or C₁₈) connected to poly(ethylene glycol) monomethyl esters (mPEG, e.g., with 6, 10 and 14 glycol units on average).

In a preferred embodiment of the use of the invention, the outer shell of the nanoparticle is hydrophilic. Particularly preferred are outer shells comprising (or consisting of) monomethyl poly(ethylene glycol) monomethylester (mPEG) chains.

In a further preferred embodiment of the use of the invention, the inner shell of the nanoparticle is nonpolar. Particularly preferred are inner shells comprising (or consisting of) long aliphatic chains. Aliphatic chains having a length of C₂-C₄₀ are even more preferred.

The core can be made of various dendritic polymers, e.g., polyglycerol, polyamide, polyamine, polyether or polyester. In a particularly preferred embodiment of the use of the present invention the dendritic core of the nanoparticle comprises (or consists of) hyperbranched poly(ethylene imine)(PEI).

In a further particularly preferred embodiment of the use of the present invention the dendritic core is functionalized with linear amphiphilic building blocks formed by alkyl diacids connected to poly(ethylene glycol) monomethylesters.

It was found that for encapsulation of Cu-ions or Zn(II)-ions a functionalization of the dendritic polymers of less than 100% is sufficient. Preferably, the degree of functionalization is 70-100%.

For the purposes of the present invention, the nanocarriers can be unimolecular or can be present in the form of distinct aggregates.

The size of the nanocarriers depends on the intended particular use. Care should be taken that the size of the nanocarriers does not inhibit clearance via the kidneys. Preferred average molecular weights of the nanocarrier are from 10,000 to 250,000 g/mol (corresponding to a average particle diameter from 3 to 7 nm).

The present invention also relates to a nanocarrier as defined above carrying metal-ions, preferably Cu-ions or Zn(II)-ions in a non-covalent encapsulated form as well as to a parmaceutical composition containing said nanocarrier and, optionally, a pharmaceutically acceptable carrier. Suitable carriers and the formulation of such pharmaceutical preparations are known to a person skilled in the art. Suitable carriers comprise e.g. phosphate-buffered common salt solutions, water, emulsions, e.g. oil/water emulsions, wetting agents, sterile solutions, etc. The pharmaceutical preparation according to the invention can be available in the form of an injection solution, tablet, ointment, suspension, emulsion, a suppository, etc. It can also be administered in the form of depots (microcapsules, zinc salts, etc.). The kind of administration of the pharmaceutical preparation depends inter alia on the kind or size of the nanocarrier which transports the active substance; it may be oral or parenteral. The methods of parenteral administration comprise the topical, intra-arterial, intra-muscular, intramedullary, intrathekal, intraventricular, intravenous, intraperitoneal, transdermal or transmucosal (nasal, vaginal, rectal, and sublingual) administration. The administration can also be made by microinjection. The suitable dose is determined by the attending physician and depends on various factors, e.g. on the patient's age, sex and weight, the kind and stage of the disease, e.g. AD, the kind of administration, etc.

Finally, the present invention relates to various uses of the nanocarriers as described above:
(a) Use of a nanocarrier as defined above carrying Cu-ions in a non-covalent encapsulated form for the preparation of a pharmaceutical composition for slowing down ageing;
(b) Use of a nanocarrier as defined above carrying Cu-ions in a non-covalent encapsulated form for the preparation of a pharmaceutical composition for the treatment of a Cu-ion transport disorder. Examples of such disorders are Alzheimer's disease (AD) or Menkes disease.

### Brief description of the drawings

Figure 1 a: Core-shell nanoparticles (CS NP) (left) and core-multi-shell nanoparticles (CMS NP) (right) and their loading with dye molecules or metal ions
   Figure 1 b: Structure of nanocarriers: CS NP (left), CMS NP (right)
Figure 2: Nanoparticle mediated uptake of Cu into SYSY cells. Treatment with CS-NP or CMS-NP slightly increased the intracellular copper concentration of human neuroblastoma SY5Y cells without pre-charging the particles with additional Cu (left, non Cu-loaded nanoparticle). Particles encapsulated Cu from normal medium.
   Preincubation of nanoparticles with additional copper led to a major increase of intracellular copper concentrations (right, Cu-loaded particle). CMS-NP were more efficient than CS-NP to increase intracellular copper levels.
Figure 3: Laser scan microscopy (LSM) using Cy3 labeled nanoparticles
Figure 4: Carrier KA82 specifically targets the nucleus of neuroblastoma SY5Y cells

The following examples illustrate the invention.

### Example 1

### General Methods

### - Metal complexation of nanocarriers

The nanoparticles were obtained by the described methods; CS NP (Krämer et al., Macromolecules 38 (2005), 8308-15, CMS NP (Radowski et al., Angew. Chem. (119) 2007, 1287-92. Isothermal titration calorimetry (ITC) and UV-Vis spectroscopic techniques were applied to prove and to quantify the encapsulation process. ITC technique has been utilized to ensue the enthalpic interactions between nanotransporters and Cu (II) ions which in turn reflect the strength and extent of metal encapsulation property of the selected nanotransporters. Encapsulation was analyzed by measuring the heat change during the titration of Cu (II) into nanotransporter solutions using MicroCal VP-ITC microcalorimeter (MicroCal, LLC, Northampton, MA). For CS NPs, the sample cell of the microcalorimeter (1.4 ml) was filled with 0.0035 mM aqueous solution of respective nanotransporters. Double-distilled water was used through out the experiment to avoid the effect of other dissolved ions. After baseline equilibration, 0.78 mM of Cu (II) was injected in 34 x 8 µL aliquots using the default injection rate. For CMS NP systems (e.g. MR 09)on the other hand, 1mM of Cu (II) solution was titrated with 0.0014 mM of nanotransporter solution. The resulting titration curves were corrected for heat of dilution of Cu²⁺ ions in water and analyzed using the Origin 7.0 ITC software supplied by MicroCal, LLC. ITC experiment measures the enthalpy change through the interaction of ligand and macromolecule and the heat released or absorbed is in direct proportion to the amount of binding between the macromolecule and the ligand. In this experiment the binding isotherm representing the interaction of Cu (II) ions to the nanotransporters are hyperbolic and for all the categories of nanotransporters, there is an initial rapid release of heat in the negative side of titration baseline indicating exothermic interaction between Cu (II) ions and the nanotransporters. The negative value of apparent change in enthalpy (*ΔHₐₚₚ*) indicates the predominant effect of charge-interaction in binding event. Fitting with one-set of binding sites mode yielded the ΔH value of -6812 Kcal/mol for CMS 10 kDa system indicating sufficiently strong encapsulation between the metal ion and the polymer. Depending on the variation of molecular architecture, saturation level of different nanotransporters by Cu (II) is reached at different molar ratio for the respective systems. The Cu (II)-CMS NP isotherms saturates at Cu (II) ions to nanotransporter molar ratio of ~40: 1.

UV-Visible spectroscopic technique also revealed the encapsulation process of the nanoparticles. An aqueous solution of the respective nanocarriers (at various concentrations calculated with the number average molecular weight, *Mₙ* value) was mixed with an aqueous solution of Cu (II) to obtain a controlled molar ratio of [Cu (II) ions/nanotransporter] between the range of 0-100. The solutions were incubated for 24h at room temperature with uniform magnetic stirring to ensure maximum encapsulation. UV-visible absorption spectra were obtained on a Scinco S-3100 spectrophotometer using 1 cm path length quartz cells. In the absence of nanoparticles with complexation capabilities, Cu (II) exists primarily as [Cu(H₂O)₆]²⁺ in aqueous solutions, which gives rise to a broad, weak absorption band at 810 nm associated with a d-d transition (ε ~ 10). In the presence of hyperbranched PEI system, λ*ₘₐₓ* for the Cu (II) d-d transition was shifted to 605 nm (ε ~ 30). In addition, a strong ligand-to-metal-charge-transfer (LMCT) transition appeared at 300 nm.

This change in UV-Vis spectrum allows following the Cu (II) ion binding with CS and CMS nanoparticles having secondary and tertiary nitrogen atoms. In this experiment, the absorbance at λ*ₘₐₓ* at 605 nm increased with increasing [Cu (II)]/[nanotransporter] ratio until a critical value is reached, above which the absorbance increased only slowly. The change in the slopes suggests different environments for the Cu (II) cation before and after this critical value and therefore the interactions between the nitrogen atoms of the nanotransporters and the metal cation. Extrapolating the two linear regions of the absorbance at this wavelength allows evaluating the maximum Cu (II)loading of the nanoparticles. This experiment indicates that both CS and CMS systems can encapsulate up to 45-50 Cu (II) ions. Binding stoichiometry as found with UV-Vis spectroscopic titration are within a considerable range of correlation with ITC data.

### - Yeast culture

*Pichia pastoris* cells were grown over night in 30 ml BMGY medium (1 % (w/v) yeast extract, 2 % (w/v) bacto peptone, 100 mM potassium phosphate, pH 6.0, 1.34 % (w/v) yeast nitrogen base, 4*10⁻⁵ % biotin, 1 % (w/v) glycerol) followed by 48 h induction in 75 ml BMMY medium [BMGY with 2 % (v/v) methanol instead of glycerol]. For SOD-activity measurements 2*10⁸ cells were harvested in the late exponential phase of growth and centrifuged at 3500 rpm for 10 min, resuspended in 1 ml of H₂O, washed once with 0.5M EDTA, pH 8.0, and then resuspended again in 1 ml of H₂O and further analyzed by SOD-activity assay. For ICP-MS measurements 15 ml of culture was centrifuged as described above. To investigate the effect of nanocarriers, induction medium was adjusted to 10µM with carrier or with carrier plus Cu(II)Cl₂, respectively.

### - Lysis of yeast cells

Washed cell pellets were resuspended in 100 µl lysis buffer (50mM potassium phosphate buffer, pH 7.4, 5 % (v/v) glycerol, 45mM MgCl2, 9*10⁻³µg/µl DNase) plus 5 µl Complete (Roche) and the equal volume of acid washed glass beads (0.5mm, Sigma) followed by eight cycles of vortexing for 30 s and alternating cooling for 30 s on ice.

### - Cell culture

Wild type CHO, SY5Y, HepG2 and HEK293 cells were cultured as described in www.lgcpromochem-atcc.com. To normalize carrier burden to the different cell lines growth rate was determined and adequate amounts of cells were disseminated in culture plates.

### - Cell lysis

Cells were washed with 1x PBS scraped from the culture plates and lysed for 30 min at 4°C in an end-over-end rotation device in lyses buffer (50mM Tris/HCl, pH 7.5, 150mM NaCl, 2mM EDTA, 2 % Trition X100, 2 % NP40). Lysis was stopped by 10 min centrifugation at 10000rpm at 4°C and supernatant was collected was further experiments.

### - Inductively coupled mass spectrometry (ICP-MS)

Washed cell pellets were analyzed by ICP-MS. ICP-MS was performed by using a HP4500 Series 300 ShieldTorch system instrument (Agilent, Waldbronn, Germany) in peak-hopping mode with spacing at 0.05 atomic mass units, three points per peak, three scans per replicate, and an integration time of 300 ms per point as described (29, 30). The rate of plasma flow was 15 liters/min with an auxiliary flow of 0.9 liters/min and a blend gas flow rate of 0.1 liters/min. The RF power was 1.2 kW. The sample was introduced by using a crossflow nebulizer at a flow rate of 1.02 liters/min. The apparatus was calibrated by using a 6.5% HNO₃ solution containing Cu and Zn at 1, 5, 10, 25, 50, 100, 200 and 400 parts per billion with Rh-103 as internal standard for all isotopes of Cu and Zn. Obtained data was normalized by the amount of yeast cells to compare the determined intracellular amount of metal ion per cell, samples were measured three times. Statistical analysis was performed using three independent measurements by calculating standard error of the mean (SEM). Statistical significance was determined by the Student's t test.

### - SOD1 assays

The assay for SOD-activity was purchased from Dojindo and is based on the highly water soluble tetrazolium salt, WST-1 (2-(4-Iodophenyl)-3-(4-nitrophenyl)-5-(2,4-disulfophenyl)-2H-tetrazolium, monosodium salt) that produces a water-soluble formazan dye upon reduction with a superoxide anion. The rate of reduction of superoxide anion is linearly related to the xanthine oxidase (XO) activity, and is inhibited by SOD. The absorbance can be measured at 450 nm. Sample preparation for the SOD activity assay (Dojindo) was done as described above but with samples diluted 1:5. Assays were performed according to the manufacturers instructions.

### - MTT-assays

MTT assays were purchased from Promega and performed in 96well plates. This assay is based on tetrazolium salt, 3-(4,5-dimethylthiazol-2-yl)-2,5diphenyltetrazolium bromide (MTT) that is taken up into cells and reduced to yield a purple formazan product which is largely impermeable to cell membranes, thus resulting in its accumulation within healthy cells. Normalized amounts of cells of the different cell lines were disseminated in 96 well plates, allowed to settle down for 48 h and then washed 3 x with 1x PBS (137mM NaCl, 2.7mM KCl, 10mM Na₂HPO₄, 2mM KH₂PO₄) followed by treatment with Optimem (Invitrogen) supplemented with several carrier and/or copper concentrations for 5 h. 20µl of CellTiter 96^{®} AQᵤₑₒᵤₛ One Solution Reagent was pipetted in each well containing 100µl culture medium. Plates were incubated 1-4 h at 37°C in a humidified, 5 % CO₂ atmosphere. Absorbance was measured at 450 nm.

### - Cell fractionation

1.8*10⁶ SY5Y were disseminated in cell culture dishes (10mm) allowed to settle down for 48 h and then washed 3 x with 1x PBS (137mM NaCl, 2.7mM KC1, 10mM Na₂HPO₄, 2mM KH₂PO₄) followed by treatment with Optimem (Invitrogen) supplemented with several carrier and/or copper concentrations for 5 h. Supernatant was collected and stored for ICP-MS analysis. Cells were washed once in 1x PBS and scraped from the plates. 2 ml PBS-T were added to incubate cells for 10 min at 4°C before homogenisation. Cell fractionation was performed by a first centrifugation step for 15 min at 500 x g resulting in P1. Followed by a second centrifugation at 2600 x g for 15 min resulting in P2. Last centrifugation was at 100000 x g for 30 min resulting in P3 and supernatant. P1, P2, P3 and supernatants were subjected to ICP-MS to analysis metal ion content of organelles.

### - Nuclei preparation

1.8*10⁶ SYSY were disseminated in cell culture dishes (10mm) allowed to settle down for 48 h and then washed 3 x with 1x PBS (137mM NaCl, 2.7mM KCl, 10mM Na₂HPO₄, 2mM KH₂PO₄) followed by treatment with Optimem (Invitrogen) supplemented with several carrier and/or copper concentrations for 5 h. 1 x PBS washed cells were treated with 1 ml Nuclei EZ lysis buffer (Sigma) and scraped from the dishes. Centrifugation at 500 x g resulted in a supernatant containing cytoplasmic components. The pellet was resuspenden in 700 µl Nuclei EZ lysis buffer (Sigma) by vortexing followed by incubation for 5 min on ice. The centrifugation procedure was repeated and nuclei and supernatant fraction were frozen to store for ICP-MS analysis.

### - Immunofluorescence

SY5Y cells were grown on glass cover slides. Images were obtained using an LSM 510 meta (Carl Zeiss) confocal microscope. Cy3 was both excited at 543 nm and detected via an 560-nm long pass filter.

### Example 2

### Cu is spontaneously encapsulated by the carrier molecules

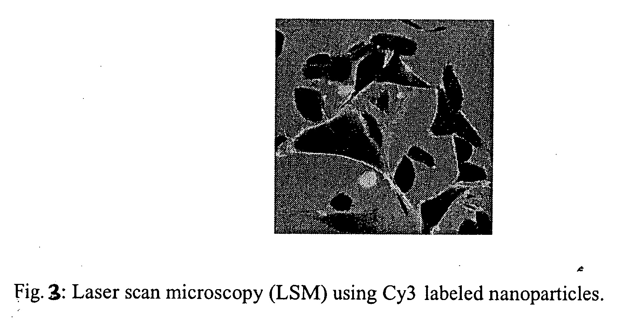

It was found that Cu is spontaneously encapsulated by the carrier molecules (NPs). Aqueous solutions of the polymer were mixed with an aqueous solution of the metal ions (Cu(II)sulfate) to obtain a controlled molar ratio [metal ions]:[polymer] of 40:1. To investigate if NPs adhere to cell surfaces, fluorescently labelled nanoparticles were used. After treatment of cells laser scan microscopy (LSM) revealed nanoparticles attached to the plasma membrane of SY5Y cells (Figure 3).

### Example 3

### Nanoparticle mediated uptake of Cu into SY5Y cells

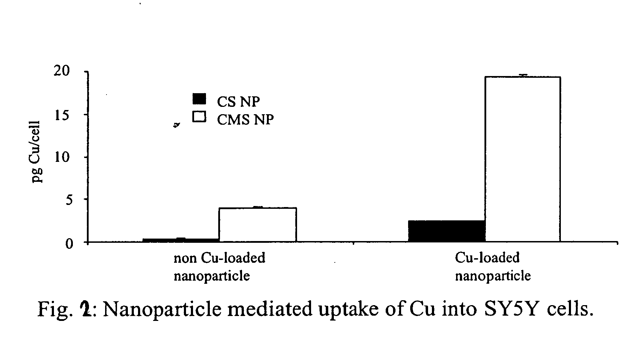

Treiber et al. (J. Biol. Chem. 279 (2004), 51858-64) showed that the overexpression of the human APP decreased intracellular Cu levels in yeast cells and has Cu-efflux activities. Yeast cells were transfected with APP and Cu content was analyzed by ICP-MS, a very sensitive method to measure Cu levels. Based on this finding, in the present experiment nanocarriers were analyzed in a cell culture system to rescue the APP induced Cu deficiency observed in the cells. The nanocarriers described in Example 1 added to the yeast culture medium drastically increased the intracellular Cu-concentration. It could be shown that CS NP as well as CMS NP both are able to transport copper into cells (Figure 1). This copper transport activity is enhanced 6-fold in the CMS NP (white bar) in comparison to CS NP (black bar) (Figure. 2). Saturation with copper led to increases of about 8 to 10 fold, respectively. These results were obtained by treatment of SY5Y cells with nanoparticles followed by ICP-MS measurements of whole cells.

Taken together, these data uncovered a novel biological function for nanotransporters in cellular import of Cu ions. Finally, preliminary experiments suggest that effective concentrations of carriers are not toxic to cells.

### Example 4

### Determination of the destination of the Cu-Cargo

To determine the destination of the Cu-cargo, cells were fractionated by differential centrifugation. Three fractions were obtained with enriched cellular compartments: (a) nuclei and cytosol; (b) mitochondria, lysomoses, and peroxisomes; and (c) plasma membrane, ER-fragments, small vesicles and microsomal fractions.

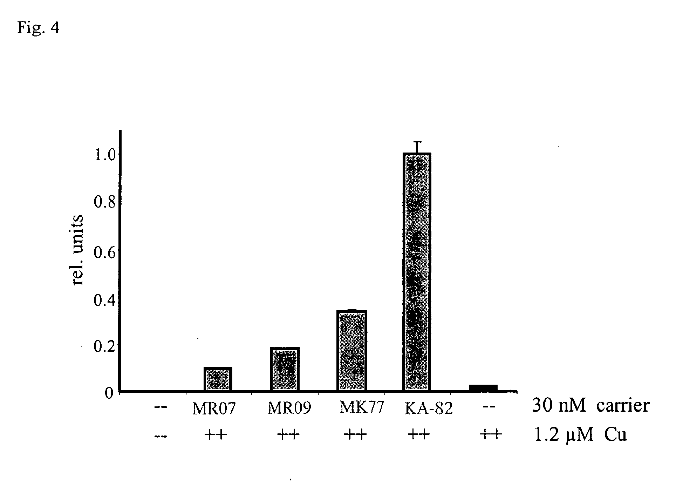

To determine how much Cu was transported to the nuclei, nuclei were isolated by using a kit system which is commercially available from Sigma Chemie (Deisenhofen, Germany) and determined the Cu-content by ICP-MS. Purified nuclei without a cytosol contaminant and subsequent ICP-MS identified KA-82 (Fig. 1b) as a nanocarrier that specifically targets the nucleus (Figure 4). The Cu-concentration in the nuclei was 44-fold increased in comparison to cells treated with free Cu. The Cu-content was 3-fold higher in comparison to the Cu-loaded carrier MK77 although both carriers were gluconolactone based. Carriers MR07 and MR09 (Fig. 1b) increased the Cu-levels in the nuclei 4- or 7.5-fold, respectively.

## Claims

1. Use of a nanocarrier having a dendritic structure which is composed of a dendritic core and at least one shell for the non-covalent encapsulation and/or transport of divalent metal-ions.

2. Use according to claim 1, wherein the nanocarrier has at least two shells.

3. Use according to claim 2, wherein the outer shell is hydrophilic.

4. Use according to claim 2 or 3, wherein the inner shell is nonpolar.

5. Use according to any one of claims 1 to 4, wherein the dendritic core comprises hyperbranched poly(ethylene imine) (PEI).

6. Use according to claim 5, wherein the core is functionalized with linear amphiphilic building blocks formed by alkyl diacids connected to poly(ethylene glycol) monomethylesters.

7. Use according to claim 6, wherein the degree of functionalization is 70-100%.

8. Use according to any one of claims 1 to 7, wherein the outer shell comprises monomethyl poly(ethylene glycol) monomethyl ester (mPEG) chains.

9. Use according to any one of claims 1 to 8, wherein the inner shell comprises long aliphatic chains.

10. Use according to claim 9, wherein the length of the aliphatic chains is C₂-C₄₀, preferably C₁₂-C₄₀.

11. Use according to any one of claims 1 to 10, wherein the average molecular weight of the nanocarrier is from 10,000 to 85,000 g/mol.

12. Use according to any one of claims 1 to 10, wherein the average particle diamter of the nanocarrier is from 3 to 7 nm.

13. Use according to any one of claims 1 to 12, wherein the divalent metal-ions are Cu-ions or Zn(II)-ions.

14. Nanocarrier as defined in any one of claims 1 to 13 carrying divalent metal-ions in a non-covalent encapsulated form.

15. Pharmaceutical composition containing a nanocarrier of claim 14.

16. Use of a nanocarrier as defined in any one of claims 1 to 13 carrying Cu-ions in a non-covalent encapsulated form for the preparation of a pharmaceutical composition for slowing down ageing.

17. Use of a nanocarrier as defined in any one of claims 1 to 13 carrying divalent metal-ions in a non-covalent encapsulated form for the preparation of a pharmaceutical composition for the treatment of a divalent metal-ion transport disorder.

18. Use of a nanocarrier as defined in any one of claims 1 to 13 carrying Cu-ions in a non-covalent encapsulated form for the preparation of a pharmaceutical composition for the treatment of a Cu-ion transport disorder.

19. Use according to claim 18, wherein the disorder is Alzheimer's disease (AD) or Menkes disease.
